(19) Europäisches Patentamt European Patent Office Office européen des brevets

(11) **EP 4 545 030 A1**

(12) **EUROPEAN PATENT APPLICATION**
published in accordance with Art. 153(4) EPC

(43) Date of publication:
**30.04.2025 Bulletin 2025/18**

(21) Application number: **22953251.0**

(22) Date of filing: **03.08.2022**

(51) International Patent Classification (IPC):
*A61B 18/14* (2006.01)     *A61N 1/36* (2006.01)
*A61N 1/05* (2006.01)     *A61B 18/00* (2006.01)

(52) Cooperative Patent Classification (CPC):
**A61B 18/00; A61B 18/14; A61N 1/05; A61N 1/36**

(86) International application number:
**PCT/KR2022/011442**

(87) International publication number:
**WO 2024/025026 (01.02.2024 Gazette 2024/05)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **26.07.2022 KR 20220092135**

(71) Applicant: **Deepqure Inc.**
**Seoul 06243 (KR)**

(72) Inventors:
• **BACH, Du Jin**
**Seoul 06243 (KR)**

• YOON, Young Il
Seoul 06243 (KR)
• JO, Seok Hyeon
Seoul 06243 (KR)
• KIM, Kee Wan
Seoul 06243 (KR)
• PARK, Chan Ho
Seoul 06243 (KR)
• PARK, Jong Sun
Seoul 06243 (KR)

(74) Representative: **Manitz Finsterwald**
**Patent- und Rechtsanwaltspartnerschaft mbB**
**Martin-Greif-Strasse 1**
**80336 München (DE)**

(54) **ELECTRODE APPARATUS FOR BLOCKING OR CONTROLLING NERVES INSIDE BODY**

(57)     An electrode apparatus for denervation or modulation in vivo includes: a main body including a shaft; an electrode unit formed to be drawn out from one end of the shaft and configured to denervate or modulate at least a part of nerves on a tube in a body; an electrode guide coupled to an end of the electrode unit and configured to guide the electrode unit to be brought into contact with the tube in the body; an electrode guide driving unit configured to move the electrode guide in forward and backward directions; and an electrode driving unit configured to move the electrode unit in the forward and backward directions in conjunction with the electrode guide driving unit. The electrode driving unit includes: a tensile force maintenance unit connected to one end of the electrode unit; and a moving unit that is connected to the tensile force maintenance unit and moves the tensile force maintenance unit in the forward and backward directions. The tensile force maintenance unit includes a lever that provides a tensile force to the electrode unit. The lever senses the tensile force with a sensor and is automatically driven in the forward and backward directions based on the sensed tensile force.

*FIG. 1*

## Description

## TECHNICAL FIELD

The present disclosure relates to an electrode apparatus for denervation or modulation in body.

## BACKGROUND

Denervation is a surgical procedure intended to control an abnormally overactive autonomic nervous system by damaging specific nerves. For example, renal denervation can treat hypertension and heart diseases by damaging renal sympathetic nerves directed to the kidney, and pulmonary denervation can treat lung diseases by damaging parasympathetic nerves directed to the lung.

Nerves usually enclose the outer walls of tubes, such as blood vessels, bronchial tubes, etc., and it may be necessary to enclose the outer walls of tubes to measure signals from the nerves or transmit electrical impulses or various energies to the nerves to damage or destroy the nerves.

For example, when a surgical procedure is performed on the renal artery, the main renal artery which is a procedure target has a diameter of from 5 mm to 7 mm, and the accessory renal artery having a diameter of from 1 mm to 2 mm may also be a procedure target. Also, the artery with distributed nerves varies in size from person to person and has different sizes depending on the location.

When the surgical procedure is performed as described above, it is important to delicately locate a component including an electrode to be formed at the end of a catheter so as to enclose the outer wall of the artery. Specifically, in order to effectively denervate or modulate the nerves, the component needs to enclose the outer wall of the artery with distributed nerves in a circumferential direction. Also, it is necessary to reliably and rapidly enclose the artery with the component including the electrode. In particular, it is important to safely and accurately attach the electrode-formed component to the outer wall of the tube in the body so as not to damage the tube in the body, which can be easily damaged by external stimuli.

## PRIOR ART DOCUMENT

## PATENT DOCUMENT

(Patent Document 1) Korean Patent Laid-open Publication No. 2013-0108401 (published on October 2, 2013)

## DISCLOSURE OF THE INVENTION

## PROBLEMS TO BE SOLVED BY THE INVENTION

The present disclosure is conceived to provide an electrode apparatus having a component that guides an electrode to enclose the circumference of a tube in the body.

Also, the present disclosure is conceived to provide an electrode apparatus configured to automatically control and safely bring a component including an electrode into close contact with an outer wall of the tube in the body without damaging the tube which can be easily damaged by external stimuli.

The problems to be solved by the present disclosure are not limited to the above-described problems. There may be other problems to be solved by the present disclosure.

## MEANS FOR SOLVING THE PROBLEMS

According to an aspect of the present disclosure, an electrode apparatus for denervation or modulation in vivo includes: a main body including a shaft; an electrode unit formed to be drawn out from one end of the shaft and configured to denervate or modulate at least a part of nerves on a tube in a body; an electrode guide coupled to an end of the electrode unit and configured to guide the electrode unit to be brought into contact with the tube in the body; an electrode guide driving unit configured to move the electrode guide in forward and backward directions; and an electrode driving unit configured to move the electrode unit in the forward and backward directions in conjunction with the electrode guide driving unit. The electrode driving unit includes: a tensile force maintenance unit connected to one end of the electrode unit; and a moving unit that is connected to the tensile force maintenance unit and moves the tensile force maintenance unit in the forward and backward directions. The tensile force maintenance unit includes a lever that provides a tensile force to the electrode unit. The lever senses the tensile force with a sensor and is automatically driven in the forward and backward directions based on the sensed tensile force.

The above-described aspects are provided by way of illustration only and should not be construed as liming the present disclosure. Besides the above-described embodiments, there may be additional embodiments described in the accompanying drawings and the detailed description.

## EFFECTS OF THE INVENTION

According to any one of the above-described means for solving the problems of the present disclosure, an electrode driving unit is located such that an electrode guide is close to a tube in the body, and then automatically and gradually brings an electrode unit into close contact with an outer wall of the tube. Thus, the electrode driving unit can automatically control and safely and accurately bring a component including an electrode into close contact with the outer wall of the tube without damaging the tube which can be easily damaged by external stimuli.

Also, the electrode driving unit can maintain the close contact state at a constant force.

## BRIEF DESCRIPTION OF THE DRAWINGS

FIG. 1 is a side view of an electrode apparatus according to an embodiment of the present disclosure.

FIG. 2 illustrates a state where an electrode guide illustrated in FIG. 1 guides and locates an electrode unit to enclose a blood vessel according to an embodiment of the present disclosure.

FIG. 3A to FIG. 3E illustrate an operation process of the electrode guide according to an embodiment of the present disclosure.

FIG. 4 is an exploded perspective view illustrating a portion of joint units illustrated in FIG. 2.

FIG. 5 is a cross-sectional view of an electrode guide driving unit located inside a main body illustrated in FIG. 1.

FIG. 6A to FIG. 6I illustrate an operation process of an electrode driving unit according to an embodiment of the present disclosure.

FIG. 7A to FIG. 7C are example diagrams provided to explain an operation process of a lever in the electrode driving unit according to another embodiment of the present disclosure.

## BEST MODE FOR CARRYING OUT THE INVENTION

Hereinafter, embodiments of the present disclosure will be described in detail with reference to the accompanying drawings to be readily implemented by a person with ordinary skill in the art to which the present invention belongs. However, it is to be noted that the present disclosure is not limited to the example embodiments but can be embodied in various other ways. In the drawings, parts irrelevant to the description are omitted in order to clearly explain the present disclosure, and like reference numerals denote like parts through the whole document.

Through the whole document, the term "connected to" or "coupled to" that is used to designate a connection or coupling of one element to another element includes both a case that an element is "directly connected or coupled to" another element and a case that an element is "electronically connected or coupled to" another element via still another element. Further, it is to be understood that the term "comprises or includes" and/or "comprising or including" used in the document means that one or more other components, steps, operation and/or existence or addition of elements are not excluded in addition to the described components, steps, operation and/or elements unless context dictates otherwise and is not intended to preclude the possibility that one or more other features, numbers, steps, operations, components, parts, or combinations thereof may exist or may be added.

Hereinafter, an embodiment of the present disclosure will be described in detail with reference to the accompanying drawings.

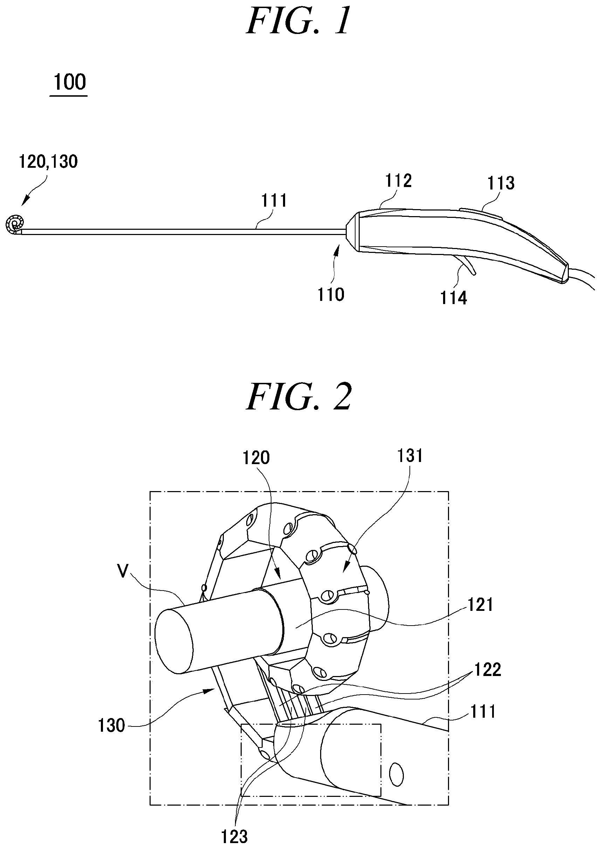

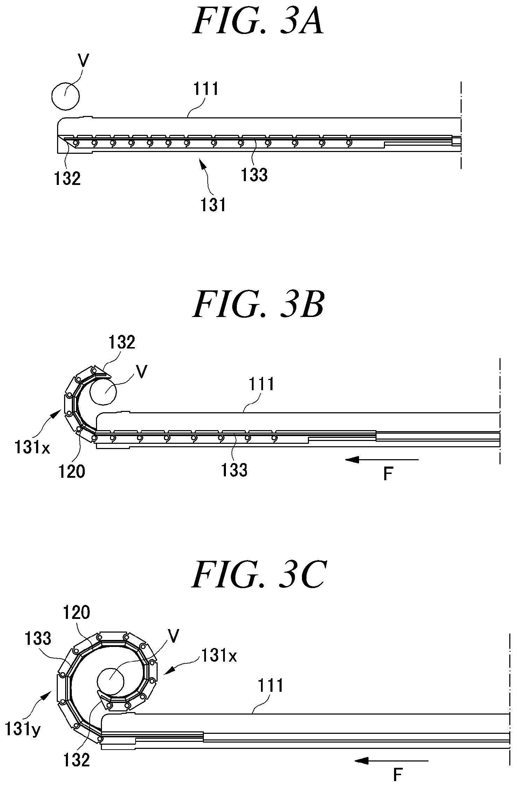

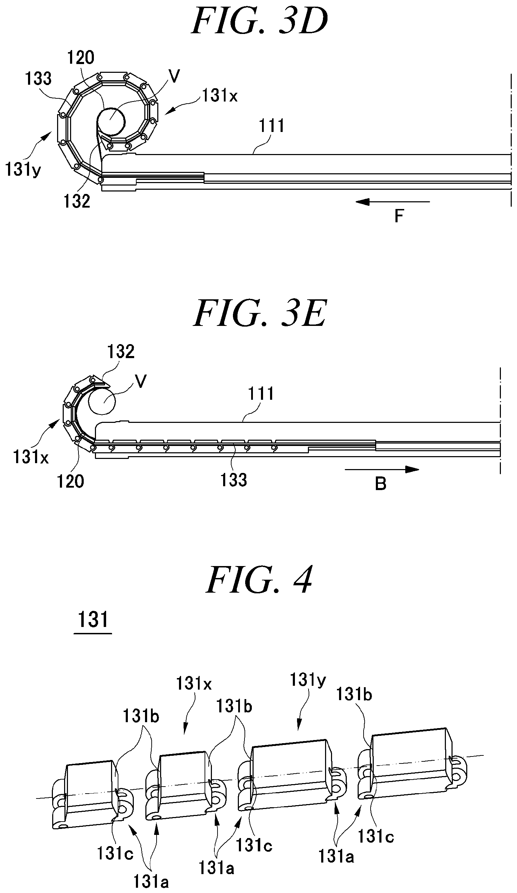

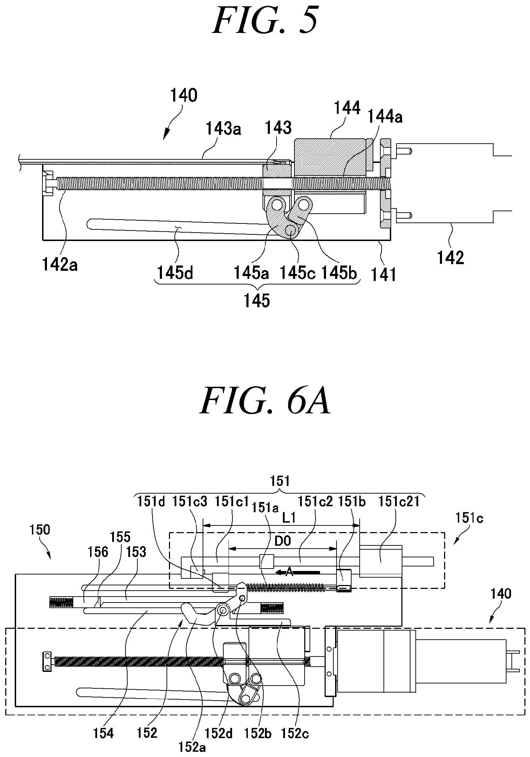

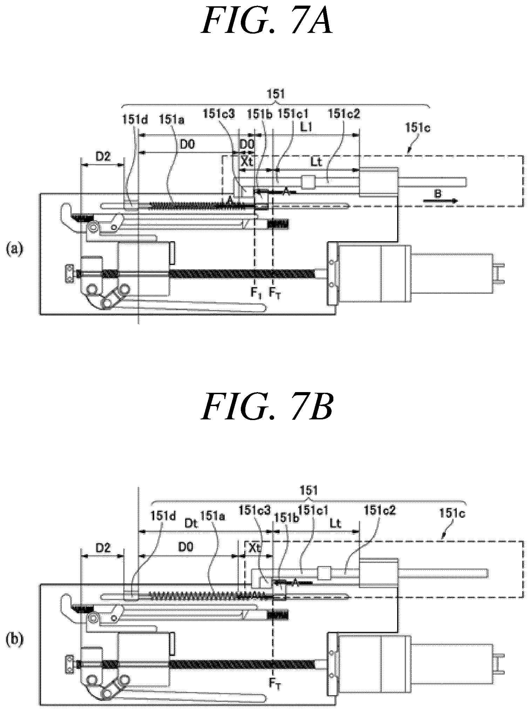

FIG. 1 is a side view of an electrode apparatus according to an embodiment of the present disclosure. FIG. 2 illustrates a state where an electrode guide illustrated in FIG. 1 guides and locates an electrode unit to enclose a blood vessel according to an embodiment of the present disclosure and FIG. 3A to FIG. 3E illustrate an operation process of the electrode guide according to an embodiment of the present disclosure. FIG. 4 is an exploded perspective view illustrating a portion of joint units illustrated in FIG. 2 and FIG. 5 is a cross-sectional view of an electrode guide driving unit located inside a main body illustrated in FIG. 1. FIG. 6A to FIG. 6I illustrate an operation process of an electrode driving unit according to an embodiment of the present disclosure and FIG. 7A to FIG. 7C are example diagrams provided to explain an operation process of a lever in the electrode driving unit according to another embodiment of the present disclosure.

Referring to FIG. 1, an electrode apparatus 100 includes a main body 110, an electrode unit 120, an electrode guide 130, and an electrode guide driving unit 140 and an electrode driving unit 150 disposed inside the main body 110.

The main body 110 may include a shaft 111 extending in one direction, a grip portion 112 connected to the shaft 111 so as to be gripped by an operator, a guide manipulation unit 113 formed on the grip portion 112 so as to manipulate an operation of the electrode guide 130, and an electrode manipulation unit 114 formed on the grip portion 112 so as to manipulate energy transfer to the electrode unit 120.

The components for driving and controlling the electrode unit 120 and the electrode guide 130 may be located inside the main body 110. For example, the electrode guide driving unit 140 configured to drive and control the electrode guide 130 and the electrode driving unit 150 configured to drive and control the electrode unit 120 may be disposed inside the main body 110.

The electrode unit 120 is formed to be drawn out from one end of the shaft 111 and configured to denervate or modulate at least a part of nerves distributed on a tissue including a tube in the body depending on manipulation by the operator. The electrode unit 120 is accommodated inside the shaft 111 and when the electrode apparatus 100 operates, the electrode unit 120 can be drawn out by means of the electrode guide 130 which will be described later.

Referring to FIG. 2, the electrode unit 120 may include a base unit 121, an electrode unit 122, and a sensor unit 123. In the electrode apparatus 100, an electrode encloses an outer surface of a tube or tube-shaped tissue V in the body and energy can be transferred through the electrode unit122. To this end, the base unit 121 may be formed as a flexible printed circuit board (PCB).

The electrode unit 122 may be composed of two electrodes extending parallel to each other on the base unit 121. The base unit 121 and the electrode unit 122 may be configured to extend and enclose the tube in the body or the like in a circumferential direction.

The electrode unit 122 may be made of a material, such as stainless steel or gold, which is harmless to the human body and conducts electricity well in order to block or denervate or control or modulate the nerves.

Also, the electrode unit 122 may transfer various types of energy from an energy source generator. For example, the energy may include radio-frequency (RF) energy, electrical energy, laser energy, ultrasonic energy, high-intensity focused ultrasound energy, cryogenic energy and other heat energy.

Also, the electrode unit 122 may be implemented as a flexible PCB for transferring RF energy, a transducer for transferring ultrasonic energy or a metal electrode for transferring high-voltage energy and thus may transfer energy to damage the nerves.

Further, the sensor unit 123 may be formed on the base unit 121. For example, the sensor unit 123 may be a thermocouple that measures a temperature by contact with the tube in the body or the like, and when neurotomy is performed with the electrode apparatus 100, the sensor unit 123 may monitor a temperature of a treatment site. As another example, the sensor unit 123 may measure signals from the nerves on the tube.

The sensor unit 123 may be, for example, a thermocouple composed of a pair of copper and constantan.

The electrode guide 130 functions to bring the electrode unit 120 into contact with the tube in the body. The electrode guide 130 is coupled to the electrode unit 120 and guides the electrode unit 120 to be deformed into a wound state where the electrode unit 120 comes into contact with the tube in the body.

Referring to **FIG. 2** to **FIG. 4,** the electrode guide 130 includes a plurality of joint units 131. The plurality of joint units 131 forms a curved winding path to enclose the circumference of the tube V in the body with the electrode unit 120 interposed therebetween. The state illustrated in **FIG. 2, FIG. 3C** and **FIG. 3D** may be a state where the plurality of joint units 131 is completely drawn out and disposed along the curved winding path.

Referring to **FIG. 3A** to **FIG. 3E,** the electrode guide 130 may further include a tip joint 132 and a wire 133. The tip joint 132 may support the electrode unit 120 and may be coupled to the end of the plurality of joint units 131 connected sequentially to each other.

The tip joint 132 may be drawn out from one end of the shaft 111 earlier than the plurality of joint units 131. As illustrated in **FIG. 3D,** the tip joint 132 may be located close to the tube V in the body and may have a tapered shape that gradually decreases in width or thickness toward the end in order to suppress interference with the electrode unit 120 or maximize the surface enclosing the tube in the body. The end of the electrode unit 120 may be fastened and fixed to the tip joint 132.

The wire 133 may be formed to sequentially penetrate the plurality of joint units 131. Referring to **FIG. 4,** each joint unit 131 may have a wire hole 131c in a longitudinal direction to allow penetration of the wire 133.

The end of the wire 133 sequentially penetrating the wire holes 131c may be coupled and fixed to the tip joint 132, and the wire 133 can slide with respect to each joint unit 131 in the wire hole 131c in the longitudinal direction.

Therefore, the wire 133 can guide the plurality of joint units 131 and the tip joint 132 to be located on the winding path and provide a force of pulling the plurality of joint units 131 and the tip joint 132 in a direction to be wound around the tube V.

The wire 133 may be operated to protrude from one end of the shaft 111 together with to the plurality of joint units 131. Here, the wire 133 may be designed to protrude less than the plurality of joint units 131 and thus can provide a force of pulling the plurality of joint units 131 along a curved path.

Each joint unit 131 may include hinge units 131a and winding support units 131b. The hinge units 131a are configured for rotatable connection to adjacent joints and may be formed on one or both sides of the joint unit 131 in the longitudinal direction in which the joint units 131 are connected parallel to each other.

As illustrated in **FIG. 4,** the hinge unit 131a may have a rotation axis in a direction intersecting the longitudinal direction so as to be connected to the hinge unit 131a of the adjacent joint unit 131. A hinge pin (not illustrated) may be inserted into and fastened to each hinge unit 131a in the direction of the rotation axis.

The winding support units 131b are configured to support the plurality of joint units 131 on the winding path and may be formed on one or both sides of the joint unit 131 in the longitudinal direction to support the adjacent joint unit 131.

As illustrated in **FIG. 2** and **FIG. 4,** the winding support unit 131b may be located adjacent to the hinge unit 131a in an inward direction of the electrode guide 130 (in a direction of winding the joint unit 131).

For example, the winding support unit 131b may be formed as a surface having a predetermined angle and area and supported by the adjacent winding support unit 131b in surface contact with each other, and, thus, a wound shape of the electrode guide 130 can be maintained.

The winding support unit 131b and the wire hole 131c may be formed at locations spaced apart from a rotation center of the hinge unit 131a in an inward direction toward the tube V in the body.

When the wire 133 is pulled backwards relative to the electrode guide 130 (when a length of the wire 133 drawn out from the shaft 111 is smaller than that of the joint units 131), a tensile force may be applied to the wire 133 in a direction of winding the electrode guide 130. On the other hand, the winding support units 131b may provide a force of supporting the joint units 131 to each other in a direction of suppressing winding of the electrode guide 130. Since the wire 133 and the winding support units 131b form a balanced force in opposite directions, the electrode guide 130 can be fixed on the winding path.

Further, the electrode guide 130 may include a first joint group 131x and a second joint group 131y. That is, the plurality of joint units 131 may be divided into the first joint group 131x and the second joint group 131y having different lengths.

Due to a difference in length, the first joint group 131x may form a first radius of curvature and the second joint group 131y may form a second radius of curvature greater than the first radius of curvature. As can be seen from **FIG. 3D**, the joint units (the first joint group 131x) having a relatively small length may form a smaller radius of curvature and the joint units (the second joint group 131y) having a relatively great length may form a greater radius of curvature.

When the joint units 131 located close to the tip joint 132 form a path having a smaller radius of curvature, a path along which the tip joint 132 enters a space between the tube in the body and the shaft 111 may be formed as shown in **FIG. 3D.** Also, the electrode guide 130 including the joint units 131 may have an overall spiral shape.

Referring to **FIG. 3A** to **FIG. 3E,** the electrode guide 130 is accommodated together with the electrode unit 120 inside the shaft 111 and may protrude from one end in a forward direction F while forming a curved winding path for surgical procedure.

For example, when the plurality of joint units 131 is sequentially drawn out, the plurality of joint units 131 may move along the curved winding path due to a difference in displacement from the wire 133 and thus may overall enclose the tube V.

Further, the electrode guide 130 is spaced apart from an outer circumferential surface of the tube and the electrode unit 120 located inside the wound electrode guide 130 may be in close contact with the outer circumferential surface of the tube V.

The plurality of joint units 131 may be drawn out from the shaft 111 by means of the electrode guide driving unit 140 and wound in a direction to enclose the tube V. Accordingly, a space where the electrode guide 130 operates can be minimized, and an operation of denervating or modulating nerves can be performed safely and accurately in a narrow space.

Referring to **FIG. 5,** the electrode guide driving unit 140 may be configured to move the electrode guide 130 in forward and backward directions, and may include a frame 141, a motor unit 142, a rod block 143, a wire block 144 and a variable connection unit 145.

The frame 141 may be provided to be fixed inside the main body and may include a guide slot or guide shaft extending in the forward and backward directions.

The motor unit 142 may be connected to the frame 141 and may rotate a rotation shaft 142a rotatably supported by the frame 141. For example, the motor unit 142 may receive electrical energy to rotate the rotation shaft 142a.

One end of the rod block 143 may be connected to the joint unit 131. The rod block 143 may be moved in the forward and backward directions by means of the motor unit 142. Specifically, the rod block 143 may be moved in the forward and backward directions in engagement with the rotation shaft 142a extending in the forward and backward directions and having a thread thereon.

The rod block 143 may include a rod 143a, which is located inside the shaft 111 and extends in one direction (forward and backward directions) and supports the joint units 131, and a corrugated component slidably coupled to the guide slot or guide shaft of the frame 141.

In addition to the above-described rotation shaft 142a and motor unit 142, the electrode guide driving unit 140 according to the present disclosure may be configured to move the rod block 143 in the forward and backward directions by various linear actuation mechanisms. For example, the electrode guide driving unit 140 may include a linear actuator of cylinder type including a pneumatic, hydraulic or electric linear actuator, or a piezoelectric or ultrasonic linear actuator.

The wire block 144 may be formed to support the wire 133 and moved in the forward and backward directions in conjunction with the rod block 143. The wire block 144 may include a corrugated component slidably inserted into the guide slot or guide shaft and a sliding hole 144a slidably accommodating the rotation shaft 142a, and may move in the forward and backward directions in parallel to the rod block 143.

The variable connection unit 145 may connect the rod block 143 and the wire block 144 to each other and vary a distance between the rod block 143 and the wire block 144. To this end, the variable connection unit 145 may include a rod link 145a, a wire link 145b, a hinge pin 145c, and a pin slot 145d.

The rod link 145a and the wire link 145b may be rotatably connected to the rod block 143 and the wire block 144, respectively. Also, the rod link 145a and the wire link 145b may be rotatably connected to each other by the hinge pin 145c.

The pin slot 145d is formed to slidably accommodate the hinge pin 145c. Specifically, the pin slot 145d is formed to extend at a predetermined tilt angle with respect to the forward and backward directions. The pin slot 145d may be formed in the frame 141.

Meanwhile, the electrode unit 120 may be drawn out from the shaft 111 by means of the electrode driving unit 150 and may be wound in the direction to enclose the tube V by means of the electrode guide 130.

Specifically, when the electrode unit 120 moves together with the electrode guide 130 in the forward direction along the curved winding path and is completely drawn out from the shaft 111 and disposed, the electrode unit 120 may be gradually brought into close contact with the tube V in the body under the control of the electrode driving unit 150. Therefore, in a state where the electrode unit 120 is stably in contact with the tube V in the body without damaging the tube V in the body, an operation of denervating or modulating nerves can be performed.

Referring to **FIG. 6A,** the electrode driving unit 150 may be configured to move the electrode unit 120 in the forward and backward directions in conjunction with the electrode guide driving unit 140. The electrode driving unit 150 may include a tensile force maintenance unit 151, a moving unit 152, a forward movement rail 153, a backward movement rail 154, a connection rail 155 connecting the forward movement rail 153 and the backward movement rail 154, and a stopper 156. For example, the forward movement rail 153 and the backward movement rail 154 may have the same length.

The tensile force maintenance unit 151 may be connected to one end of the electrode unit 120 and may provide a tensile force to the electrode unit 120. The tensile force maintenance unit 151 may include a spring 151a, a protrusion 151b upwardly protruding from one side, a lever 151c, and an electrode connection portion 151d on the other side.

The protrusion 151b may be moved in the backward direction by the lever 151c, and as described below, the spring 151a may provide a tensile force to the electrode unit 120 due to backward movement of the lever 151c.

The lever 151c may generate the tensile force by extending the spring 151a. Specifically, the lever 151c may sense the tensile force generated in the spring 151a with a sensor. The lever 151c may be automatically driven in the forward and backward directions based on the sensed tensile force.

Referring to **FIG. 6A,** the lever 151c may include a link 151c1, an actuator 151c2, and a force sensor 151c3.

After forward movement of the electrode driving unit 150 and the electrode guide driving unit 140 is completed, the lever 151c may move the protrusion 151b in the backward direction by means of the link 151c1 to increase a length of the spring 151a.

Herein, the link 151c1 may move the protrusion 151b in the backward direction to generate a tensile force of the spring 151a. Herein, one side of the link 151c1 may be connected to the actuator 151c2 and the other side may fix the force sensor 151c3.

As illustrated in **FIG. 6A,** the link 151c1 may extend in the longitudinal direction. For example, the link 151c1 may extend in a "¬" shape to be in contact with a side surface of the protrusion 151b and thus to control movement of the protrusion 151b. The link 151c2 according to the present disclosure may have various shapes, and is not limited to the embodiment described herein.

For example, the lever 151c may drive the link 151c1 in the forward and backward directions. Herein, the link 151c1 may operate by a linear sliding mechanism. The lever 151c may move the protrusion 151b in the forward and backward directions while moving the link 151c1 in the forward and backward directions.

The force sensor 151c3 may be formed in an inner surface of the link 151c1 in contact with the protrusion 151b. For example, the force sensor 151c3 may be located on a surface where the link 151c1 abuts against the protrusion 151b. Herein, the force sensor 151c3 may be a load cell.

The force sensor 151c3 formed in the inner surface of the link 151c1 may sense the tensile force generated in the spring 151a. Specifically, the link 151c1 including the force sensor 151c3 in the inner surface may move the protrusion 151b and generate the tensile force in the spring 151a while moving in the forward and backward directions. Herein, the force sensor 151c3 abutting against the protrusion 151b may sense the tensile force generated in the spring 151a.

Meanwhile, after the tensile force is generated, the force sensor 151c3 may also sense a restoring force of the spring 151a. That is, the force sensor 151c3 abutting against a side surface of the protrusion 151b may sense the tensile force or restoring force of the spring 151a through the protrusion 151b.

The actuator 151c2 may be connected to one side of the link 151c1. The actuator 151c2 may drive the link 151c1 in the forward and backward directions based on the tensile force of the spring 151a sensed by the force sensor 151c3. For example, the actuator 151c2 may be a linear actuator and may operate by a linear sliding mechanism.

Specifically, when the restoring force of the spring 151a sensed by the force sensor 151c3 is smaller than the generated tensile force, the actuator 151c2 may drive the link 151c1 in the backward direction. Meanwhile, when the restoring force of the spring 151a sensed by the force sensor 151c3 is greater than the generated tensile force, the actuator 151c2 may drive the link 151c1 in the forward direction.

That is, the lever 151c may sense the tensile force or restoring force of the spring 151a through the force sensor 151c3 and move the link 151c1 in the forward and backward directions based on the sensed tensile force or restoring force of the spring 151a. Accordingly, the electrode unit 120 can be automatically brought into close contact with an outer wall of the tube V more accurately and safely, and can be controlled in real time to be maintained at a constant force.

According to the present disclosure, after the tensile force maintenance unit 151 and the moving unit 152 are disconnected from each other, the tensile force of the spring 151a is gradually transferred to the electrode unit 120 by automatically driving the lever 151c. Therefore, the electrode unit 120 can be safely brought into close contact with the tube V.

The electrode connection portion 151d may be connected to one side of the electrode unit 120 and may transfer the tensile force of the spring 151a to the electrode unit 120. For example, as the protrusion 151b is moved in the backward direction by the link 151c1, the electrode unit 120 may be brought into contact with the tube V.

The moving unit 152 may move the tensile force maintenance unit 151 in the forward direction until the electrode guide 130 encloses the circumference of the tube V in the body in a state where the moving unit 152 is connected to the tensile force maintenance unit 151, and then may be disconnected from the tensile force maintenance unit 151.

The moving unit 152 may include a connection portion 152a for connection to the tensile force maintenance unit 151, a pin 152b, a support 152c, and a hinge 152d.

The pin 152b may be formed on one side of the connection portion 152a, and may move in the forward direction along the forward movement rail 153 or may move in backward direction along the backward movement rail 154. Accordingly, the moving unit 152 may move in the forward direction together with the tensile force maintenance unit 151 along the forward movement rail 153 through the pin 152b, and may move in the backward direction along the backward movement rail 154 after being disconnected from the tensile force maintenance unit 151.

The support 152c may be connected to the electrode guide driving unit 140. For example, the support 152c may be connected to the wire block 144.

The hinge 152d is configured to make the connection portion 152a rotate, and when the pin 152b moves from the forward movement rail 153 to the connection rail 155, the hinge 152d rotates, and, thus, the connection portion 152a may be disconnected from the tensile force maintenance unit 151. Therefore, after the connection portion 152a is disconnected from the tensile force maintenance unit 151, each of the electrode unit 120 and the electrode guide 130 may move.

When the pin 152b moves in the backward direction, the stopper 156 may suppress the pin 152b not to move again along the connection rail 155. The stopper 156 may block the connection rail 155 when the pin 152b is located on the backward movement rail 154 through the connection rail 155.

Hereafter, driving of the electrode unit 120 by means of the electrode driving unit 150 will be described with reference to **FIG. 6A** to **FIG. 6I**. **FIG. 6A** to **FIG. 6I** illustrate states corresponding to the states illustrated in **FIG. 3A** to **FIG. 3E**.

The electrode driving unit 150 and the electrode guide driving unit 140 illustrated in **FIG. 6A** may be in a state right before forward movement starts or right after backward movement ends. Therefore, as illustrated in **FIG. 3A**, the electrode unit 120 and the electrode guide 130 may be in a state right before enclosing the circumference of the tube V in the body or right after the electrode unit 120 and the electrode guide 130 having enclosed the circumference of the tube V in the body are transitioned to the state before enclosing the tube V. That is, the electrode unit 120 and the electrode guide 130 may be in a state right before or right after neurotomy is performed with the electrode apparatus 100.

Herein, a force F applied to the protrusion 151b in a forward direction A can be represented by the following Equation 1.

<Equation 1>

$$F = kX_0 = 0 = F_0$$

In Equation 1, k is the spring constant and X is the spring elongation length and may be denoted by $X_0$, $X_1$, and $X_2$. That is, a force $F_0$ applied to the protrusion 151b is "0".

Therefore, a length between one side of the electrode connection portion 151d and the protrusion 151b may be, for example, "D0" in which the spring 151a does not change in length. Also, a length between the force sensor 151c3 and one side 151c21 of an actuator 152c2 may be, for example, "L1" in which the spring 151a does not change in length. Further, the force F measured by the force sensor 151c3 may be "0".

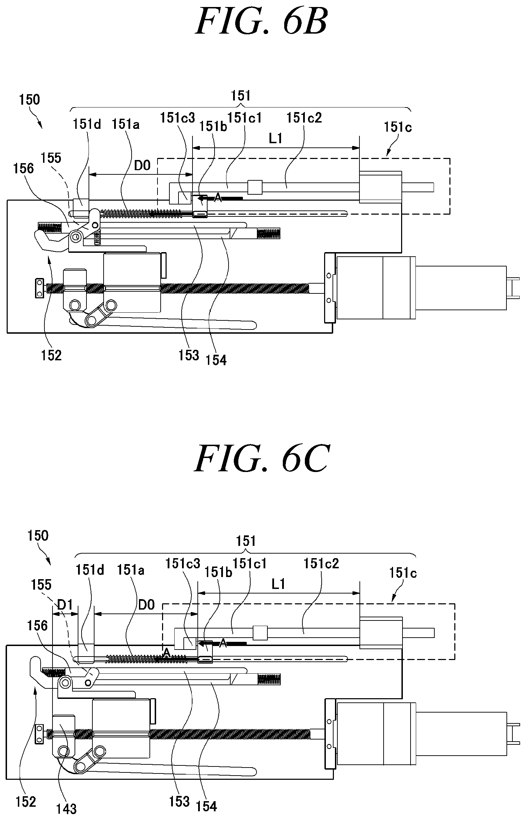

Referring to **FIG. 6B,** the electrode driving unit 150 may move in the forward direction along a path provided by the forward movement rail 153 together with the electrode guide driving unit 140 moving in the forward direction. As illustrated in **FIG. 3B** and **FIG. 3C,** due to forward movement of the electrode driving unit 150 and the electrode guide driving unit 140, the electrode unit 120 and the electrode guide 130 may be drawn out from the shaft 111 in the forward direction F and wound to enclose the circumference of the tube V in the body.

Specifically, when the electrode guide driving unit 140 is moved in the forward direction by driving the motor unit 142, the tensile force maintenance unit 151 is also moved in the forward direction through the moving unit 152.

That is, as the electrode guide driving unit 140 moves in the forward direction, the pin 152b of the moving unit 152 connected to the electrode guide driving unit 140 may move in the forward direction along the forward movement rail 153. Here, the electrode guide 130 is drawn out from the shaft 111 in the forward direction F and the tensile force maintenance unit 151 connected to the moving unit 152 moves in the forward direction, and, thus, the electrode unit 120 of which one end is connected to the electrode connection portion 151d may also be drawn out from the shaft 111.

Herein, the force $F_0$ applied to the protrusion 151b in the forward direction A is "0", and the length between one side of the electrode connection portion 151d and the protrusion 151b may be "D0" in which the spring 151a does not change in length. Also, the length between the force sensor 151c3 and the one side 151c21 of the actuator 152c2 may be "L1" in which the spring 151a does not change in length. Further, the force F measured by the force sensor 151c3 may be "0".

Referring to **FIG. 6C,** when forward movement of the electrode guide driving unit 140 is completed, the pin 152b of the moving unit 152 moves along the connection rail 155 and the hinge 152d rotates. Thus, the connection portion 152a and the tensile force maintenance unit 151 may be disconnected from each other.

When the electrode guide driving unit 140 and the pin 152b of the moving unit 152 move to the end of the path provided by the forward movement rail 153, the electrode unit 120 and the electrode guide 130 may be together wound to be close to the tube V in the body as illustrated in **FIG. 3C.** Here, the electrode guide 130 may be in a state where the plurality of joint units 131 is completely drawn out along the curved winding path.

Herein, the force F0 applied to the protrusion 151b in the forward direction A is "0", and the length between one side of the electrode connection portion 151d and the protrusion 151b may be "D0" in which the spring 151a does not change in length. Also, the length between the force sensor 151c3 and the one side 151c21 of the actuator 152c2 may be "L1" in which the spring 151a does not change in length. Further, the force F measured by the force sensor 151c3 may be "0".

Meanwhile, as the moving unit 152 and the tensile force maintenance unit 151 are disconnected from each other, a length between the rod block 143 of the electrode guide driving unit 140 and the other side of the electrode connection portion 151d may be increased to "D1".

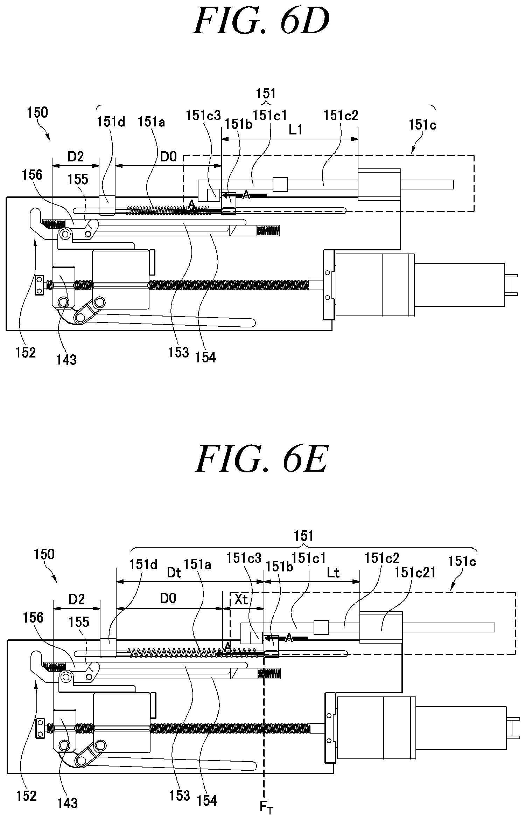

Referring to **FIG. 6D** and **FIG. 6E,** after the tensile force maintenance unit 151 is disconnected from the moving unit 152, the protrusion 151b may be moved in the backward direction by the link 151c1 of the lever 151c. Referring to **FIG. 6D,** as the tensile force maintenance unit 151 is disconnected from the moving unit 152, the tensile force maintenance unit 151 can continue to move in the backward direction. Therefore, the length between the rod block 143 of the electrode guide driving unit 140 and the other side of the electrode connection portion 151d may be further increased to "D2". Herein, the increased length "D2" may be in inverse proportion to the diameter of the tube V in contact with the electrode unit 120. Herein, the force F0 applied to the protrusion 151b in the forward direction A is "0", and the length between one side of the electrode connection portion 151d and the protrusion 151b may be "D0" in which the spring 151a does not change in length. Also, the length between the force sensor 151c3 and the one side 151c21 of the actuator 152c2 may be "L1" in which the spring 151a does not change in length. Further, the force F measured by the force sensor 151c3 may be "0".

Due to the increased length D2, the electrode unit 120 may be brought into contact with the tube V in the body without a change in length of the spring 151a as illustrated in **FIG. 3D.**

Referring to **FIG. 6E,** the link 151c1 may move the protrusion 151b in the backward direction by driving the lever 151c and increase the length of the spring 151a by a predetermined length Xt.

Specifically, the lever 151c may drive the link 151c1 in the backward direction by means of the actuator 151c2. For example, the length of the link 151c1 may decrease to the length, *i.e.,* "Lt", between the force sensor 151c3 and the one side 151c21 of the actuator 152c2.

Since the link 151c1 moves in the backward direction as the actuator 152c2 is driven, the protrusion 151b may also be moved in the backward direction and the length of the spring 151a connected to the protrusion 151b may increase by the predetermined length Xt.

**That** is, as shown in **FIG. 6E,** after the tensile force maintenance unit 151 is disconnected from the moving unit 152, the protrusion 151b is moved in the backward direction by the link 151c1, and, thus, the length of the spring 151a may increase by the predetermined length Xt. Also, as shown in **FIG. 3D,** as a tensile force is provided to the electrode unit 120, the electrode unit 120 can be brought into close contact with the tube V in the body with a predetermined tensile force.

Herein, the force F applied to the protrusion 151b in the forward direction A can be represented by the following Equation 2.

<Equation 2>

$$F_T = kX_t$$

Referring to Equation 2, the force F measured by the force sensor 151c3 may be a tensile force FT which is generated as the length of the spring 151a increases by the predetermined length Xt.

Further, the length between one side of the electrode connection portion 151d and the protrusion 151b may be "DT (=D0+Xt)" when the length of the spring 151a increases.

The electrode unit 120 brought into close contact with the tube V in the body with a predetermined tensile force may transfer energy for damaging nerves to enable neurotomy.

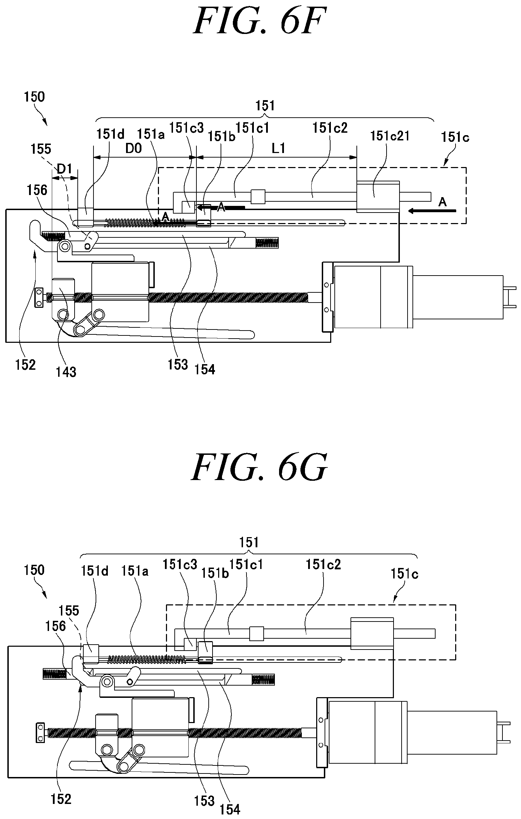

Then, referring to **FIG. 6F,** when the link 151c1 of the lever 151c returns back to its original position, the spring 151a may returns back to its original state.

Specifically, when the link 151c1 returns back to its original position by manipulating the lever 151c, the protrusion 151b also returns back to its original position. Thus, the length of the spring 151a may decrease again to a predetermined length (D2->D1). The actuator 151c2 may move the link 151c1 in the forward direction A.

Therefore, all the tensile force generated in the spring 151a is removed, and, thus, the electrode unit 120 which is spaced apart from the electrode guide 130 and in close contact with the tube V in the body may be attached again to the electrode guide 130 as illustrated in **FIG. 3C**.

Herein, the force F0 applied to the protrusion 151b in the forward direction A is "0", and the length between one side of the electrode connection portion 151d and the protrusion 151b may be "D0" in which the spring 151a does not change in length. Also, the length between the force sensor 151c3 and the one side 151c21 of the actuator 152c2 may be "L1" in which the spring 151a does not change in length.

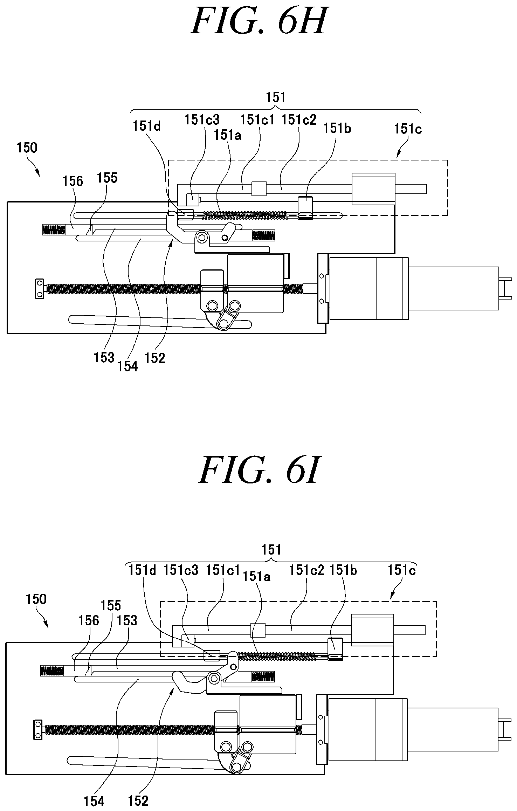

Referring to **FIG. 6G** and **FIG. 6H,** after the protrusion 151b and the link 151c1 return back to their original positions, the moving unit 152 may move in the backward direction. Since the moving unit 152 and the electrode guide driving unit 140 move in the backward direction along the backward movement rail 154, it is possible to make the electrode guide 130 deviate from the circumference of the tube V in the body as illustrated in **FIG. 3E.**

Specifically, as the electrode guide driving unit 140 moves in the backward direction, the pin 152b of the moving unit 152 connected to the electrode guide driving unit 140 may move in the backward direction along the backward movement rail 154. Thus, the other side of the connection portion 152a meets the electrode connection portion 151d of the tensile force maintenance unit 151, which causes the tensile force maintenance unit 151 to move in the backward direction.

When the pin 152b moves in the backward direction, the electrode driving unit 150 blocks the connection rail 155 by means of the stopper 156 to suppress the pin 152b not to move again along the connection rail 155. For example, the stopper 156 may include a spring that compresses the stopper 156 in order for the pin 152b to move along the connection rail 155 and returns the stopper 156 back to its original state when the pin 152b is located on the backward movement rail 154.

**As** the electrode guide driving unit 140 and the electrode driving unit 150 move in the backward direction, the electrode unit 120 and the electrode guide 130 may move in a backward direction B toward the shaft 111 as illustrated in **FIG. 3E.**

When backward movement of the electrode guide driving unit 140 and the electrode driving unit 150 is completed, the pin 152b of the moving unit 152 may be located on the forward movement rail 153, *i.e.,* in a standby state, as illustrated in **FIG. 6I.** Here, the electrode unit 120 and the electrode guide 130 may also be in a standby state before being drawn out from the shaft 111 as illustrated in **FIG. 3A.**

Referring to **FIG. 7A to FIG. 7C,** even after the electrode unit 120 is brought into close contact with the tube V, the lever 151c can automatically regulate forward or backward driving of the electrode unit 120. Thus, the electrode unit 120 can be brought into close contact with the tube V in the body with a predetermined tensile force, and the predetermined tensile force may be arbitrarily regulated depending on the diameter and elasticity of the tube V.

**FIG. 7B** illustrates an example where after the tensile force maintenance unit 151 and the moving unit 152 are disconnected from each other, the protrusion 151b is moved in the backward direction by the lever 151c. In this case, the lever 151c may sense the tensile force or restoring force of the spring 151a through the force sensor 151c3.

**FIG. 7A** illustrates an example where a restoring force F1 of the spring 151a sensed by the force sensor 151c3 as illustrated in **FIG. 7B** is smaller than tensile force FT which is generated as the length of the spring 151a increases. In this case, the lever 151c may move the link 151c1 in the backward direction B through the actuator 151c2.

That is, the lever 151c may move the link 151c1 in the backward direction B and thus move the protrusion 151b in the backward direction. As the protrusion 151b is moved in the backward direction, a tensile force may be added to the spring 151a.

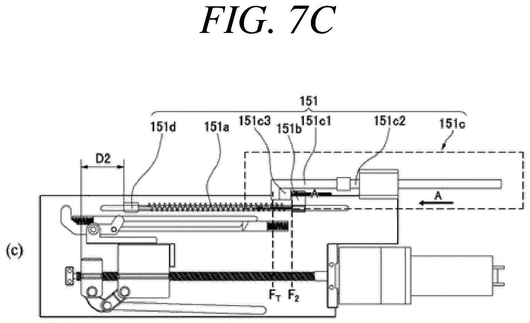

**FIG. 7C** illustrates an example where the restoring force F1 of the spring 151a sensed by the force sensor 151c3 as illustrated in **FIG. 7B** is greater than tensile force FT which is generated as the length of the spring 151a increases. In this case, the lever 151c may move the link 151c1 in the forward direction A through the actuator 151c2.

That is, the lever 151c may move the link 151c1 in the forward direction A and thus move the protrusion 151b in the forward direction. As the protrusion 151b is moved in the forward direction, a tensile force may be removed from the spring 151a.

**As** shown in **FIG. 7A, FIG. 7B,** and **FIG. 7C,** according to the present disclosure, the electrode unit 120 can be controlled in real time to be in close contact with the tube V with a predetermined tensile force based on the tensile force or restoring force of the spring 151a sensed by the force sensor 151c3 in the lever 151c, and the predetermined tensile force may be arbitrarily regulated depending on the diameter and elasticity of the tube V.

Accordingly, neurotomy can be performed in a state where the electrode unit 120 is in close contact with the tube V in the body accurately and safely.

The above description of the present disclosure is provided for the purpose of illustration, and it would be understood by a person with ordinary skill in the art to which the present disclosure belongs that various changes and modifications may be made without changing technical conception and essential features of the present disclosure. Thus, it is clear that the above-described examples are illustrative in all aspects and do not limit the present disclosure. For example, each component described to be of a single type can be implemented in a distributed manner, likewise, components described to be distributed can be implemented in a combined manner.

The scope of the present disclosure is defined by the following claims rather than by the detailed description of the embodiment, and it should be understood that all modifications and embodiments conceived from the meaning and scope of the claims and their equivalents are included in the scope of the present disclosure.

## Claims

1. An electrode apparatus for denervation or modulation in vivo, comprising:

   a main body including a shaft;
   an electrode unit formed to be drawn out from one end of the shaft and configured to denervate or modulate at least a part of nerves on a tube in a body;
   an electrode guide coupled to an end of the electrode unit and configured to guide the electrode unit to be brought into contact with the tube in the body;
   an electrode guide driving unit configured to move the electrode guide in forward and backward directions; and
   an electrode driving unit configured to move the electrode unit in the forward and backward directions in conjunction with the electrode guide driving unit,
   wherein the electrode driving unit includes:

   a tensile force maintenance unit connected to one end of the electrode unit; and
   a moving unit that is connected to the tensile force maintenance unit and moves the tensile force maintenance unit in the forward and backward directions,
   the tensile force maintenance unit includes a lever that provides a tensile force to the electrode unit, and
   the lever senses the tensile force with a sensor and is automatically driven in the forward and backward directions based on the sensed tensile force.

2. The electrode apparatus of Claim 1, wherein the tensile force maintenance unit further includes:

   a spring that generates the tensile force, and
   the lever generates the tensile force by extending the spring and provides the generated tensile force to the electrode unit.

3. The electrode apparatus of Claim 2, wherein the moving unit moves the tensile force maintenance unit in the forward direction until the electrode guide encloses the circumference of the tube in the body in a state where the moving unit is connected to the tensile force maintenance unit, and then is disconnected from the tensile force maintenance unit.

4. The electrode apparatus of Claim 3, wherein the tensile force maintenance unit further includes:

   a protrusion protruding from one side, and
   the lever includes:

   a link that moves the protrusion in the backward direction to generate the tensile force of the spring;
   a force sensor that is formed in an inner surface of the link in contact with the protrusion and configured to sense the tensile force generated in the spring; and
   an actuator that drives the link in the forward and backward directions based on the sensed tensile force of the spring.

5. The electrode apparatus of Claim 4,

   wherein the actuator drives the link in the backward direction when a restoring force of the spring sensed by the force sensor is smaller than the tensile force, and
   the actuator drives the link in the forward direction when the restoring force of the spring sensed by the force sensor is greater than the tensile force.

6. The electrode apparatus of Claim 4, wherein the electrode unit is brought into contact with the tube as the protrusion is moved in the backward direction by the link.

7. The electrode apparatus of Claim 3, wherein the moving unit further includes:

   a connection portion for connection to the tensile force maintenance unit; and
   a pin that is formed on the connection portion and used to move the tensile force maintenance unit in the forward direction by the moving unit, and
   the electrode driving unit further includes:
   a forward movement rail along which the pin moves in the forward direction.

8. The electrode apparatus of Claim 7,

   wherein the electrode driving unit further includes:
   a backward movement rail along which the pin moves in the backward direction in order for the moving unit to make the electrode guide deviate from the circumference of the tube in the body after the moving unit is disconnected from the tensile force maintenance unit.

9. The electrode apparatus of Claim 8, wherein the moving unit further includes:

a support connected to the electrode guide driving unit; and
a hinge unit configured to make the connection portion rotate,
the electrode driving unit further includes:

a connection rail connecting the forward movement rail and the backward movement rail, and
when the pin moves along the connection rail, the hinge unit rotates and the connection portion is disconnected from the tensile force maintenance unit.

10. The electrode apparatus of Claim 9, wherein the electrode driving unit further includes:
a stopper that blocks the connection rail when the pin is located on the backward movement rail through the connection rail in order to suppress the pin not to move again along the connection rail when the pin moves in the backward direction.

# FIG. 1

# FIG. 2

## FIG. 3A

## FIG. 3B

## FIG. 3C

## FIG. 3D

## FIG. 3E

## FIG. 4

## FIG. 5

## FIG. 6A

## FIG. 6B

## FIG. 6C

# FIG. 6D

# FIG. 6E

*FIG. 6F*

*FIG. 6G*

## FIG. 6H

## FIG. 6I

# FIG. 7A

(a)

# FIG. 7B

(b)

# FIG. 7C

## INTERNATIONAL SEARCH REPORT

| International application No. |
|---|
| **PCT/KR2022/011442** |

| A. | CLASSIFICATION OF SUBJECT MATTER |
|---|---|

**A61B 18/14**(2006.01)i; **A61N 1/36**(2006.01)i; **A61N 1/05**(2006.01)i; **A61B 18/00**(2006.01)i

According to International Patent Classification (IPC) or to both national classification and IPC

| B. | FIELDS SEARCHED |
|---|---|

Minimum documentation searched (classification system followed by classification symbols)

A61B 18/14(2006.01); A61B 10/02(2006.01); A61B 18/00(2006.01); A61B 18/04(2006.01); A61B 34/00(2016.01); A61F 2/00(2006.01); A61M 1/10(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 수술(surgery), 신경(nerve), 차단(block), 샤프트(shaft), 전극(electrode), 장력 (tension)

| C. | DOCUMENTS CONSIDERED TO BE RELEVANT |
|---|---|

| Category* | Citation of document, with indication, where appropriate, of the relevant passages | Relevant to claim No. |
|---|---|---|
| Y | KR 10-2373974 B1 (DEEPQURE INC.) 15 March 2022 (2022-03-15)<br>See claims 1-10. | 1-10 |
| Y | KR 10-2020-0050227 A (KOREA ADVANCED INSTITUTE OF SCIENCE AND TECHNOLOGY) 11 May 2020 (2020-05-11)<br>See paragraphs [0056]-[0057]. | 1-10 |
| A | US 2021-0401488 A1 (ETHICON, INC.) 30 December 2021 (2021-12-30)<br>See paragraphs [0032]-[0067]; and figures 3-9. | 1-10 |
| A | US 2014-0371855 A1 (CLEMENT, Claude et al.) 18 December 2014 (2014-12-18)<br>See claims 47-92; and figures 1-7. | 1-10 |
| A | KR 10-2015-0021632 A (KOREA INSTITUTE OF MACHINERY & MATERIALS) 03 March 2015 (2015-03-03)<br>See paragraphs [0025]-[0063]; and figures 4-13. | 1-10 |

☐ Further documents are listed in the continuation of Box C.   ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
|---|---|
| **20 April 2023** | **20 April 2023** |

| Name and mailing address of the ISA/KR | Authorized officer |
|---|---|
| **Korean Intellectual Property Office**<br>**Government Complex-Daejeon Building 4, 189 Cheongsa-**<br>**ro, Seo-gu, Daejeon 35208** | |
| Facsimile No. **+82-42-481-8578** | Telephone No. |

Form PCT/ISA/210 (second sheet) (July 2022)

**INTERNATIONAL SEARCH REPORT**
Information on patent family members

| International application No. |
| --- |
| **PCT/KR2022/011442** |

| Patent document cited in search report | | | Publication date (day/month/year) | Patent family member(s) | | | Publication date (day/month/year) |
| --- | --- | --- | --- | --- | --- | --- | --- |
| KR | 10-2373974 | B1 | 15 March 2022 | WO | 2023-022253 | A1 | 23 February 2023 |
| KR | 10-2020-0050227 | A | 11 May 2020 | KR | 10-2206393 | B1 | 22 January 2021 |
| US | 2021-0401488 | A1 | 30 December 2021 | CN | 105744907 | A | 06 July 2016 |
| | | | | CN | 110575244 | A | 17 December 2019 |
| | | | | EP | 3071134 | A1 | 28 September 2016 |
| | | | | JP | 2016-538054 | A | 08 December 2016 |
| | | | | JP | 6529971 | B2 | 12 June 2019 |
| | | | | KR | 10-2016-0088393 | A | 25 July 2016 |
| | | | | US | 11141214 | B2 | 12 October 2021 |
| | | | | US | 2015-0141810 | A1 | 21 May 2015 |
| | | | | US | 2020-0000514 | A1 | 02 January 2020 |
| | | | | WO | 2015-077093 | A1 | 28 May 2015 |
| US | 2014-0371855 | A1 | 18 December 2014 | CN | 104144656 | A | 12 November 2014 |
| | | | | CN | 104144656 | B | 22 February 2017 |
| | | | | EP | 2793743 | A1 | 29 October 2014 |
| | | | | EP | 2793743 | B1 | 04 November 2020 |
| | | | | JP | 2015-500721 | A | 08 January 2015 |
| | | | | JP | 2018-020164 | A | 08 February 2018 |
| | | | | JP | 6532921 | B2 | 19 June 2019 |
| | | | | KR | 10-2014-0119036 | A | 08 October 2014 |
| | | | | US | 10441399 | B2 | 15 October 2019 |
| | | | | US | 11246695 | B2 | 15 February 2022 |
| | | | | US | 2017-0290649 | A1 | 12 October 2017 |
| | | | | US | 2020-0046480 | A1 | 13 February 2020 |
| | | | | US | 9717579 | B2 | 01 August 2017 |
| | | | | WO | 2013-091730 | A1 | 27 June 2013 |
| | | | | WO | 2013-093074 | A1 | 27 June 2013 |
| KR | 10-2015-0021632 | A | 03 March 2015 | KR | 10-1551311 | B1 | 08 September 2015 |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

• KR 20130108401